# EUROPEAN PATENT SPECIFICATION

(11) **EP 3 366 242 B1**
(45) Date of publication and mention of the grant of the patent: **22.12.2021**
(21) Application number: 18156239.8
(22) Date of filing: 12.02.2018
(51) Int. Cl.: A61B 17/70

(54) **SPINAL IMPLANT SYSTEM**
WIRBELSÄULENIMPLANTATSYSTEM
SYSTÈME D'IMPLANT VERTÉBRAL

(30) Priority: 24.02.2017 US 201715441713
(43) Date of publication of application: 29.08.2018
(73) Proprietor: Warsaw Orthopedic, Inc., Warsaw, IN 46582 (US)
(72) Inventor: BOBBITT, Dustin, Olive Branch, MS Mississippi 38654 (US); ARMSTRONG, Rex W., Cordova, TN Tennessee 38016-8434 (US)
(74) Representative: Viering, Jentschura & Partner mbB Patent- und Rechtsanwälte

(56) References cited:
- US-A1- 2007 123 860
- US-A1- 2013 211 458
- US-A1- 2016 166 289
- US-B1- 6 749 612
- US-B2- 7 909 854

## Description

### TECHNICAL FIELD

The present disclosure generally relates to medical devices for the treatment of musculoskeletal disorders, and more particularly to a spinal implant system for treating a spine.

### BACKGROUND

Spinal pathologies and disorders such as scoliosis and other curvature abnormalities, kyphosis, degenerative disc disease, disc herniation, osteoporosis, spondylolisthesis, stenosis, tumor and fracture may result from factors including trauma, disease and degenerative conditions caused by injury and aging. Spinal disorders typically result in symptoms including deformity, pain, nerve damage, and partial or complete loss of mobility.

Non-surgical treatments, such as medication, rehabilitation and exercise can be effective, however, may fail to relieve the symptoms associated with these disorders. Surgical treatment of these spinal disorders includes correction, fusion, fixation, discectomy, laminectomy and implantable prosthetics. As part of these surgical treatments, spinal constructs including vertebral rods are often used to provide stability to a treated region. Rods redirect stresses away from a damaged or defective region while healing takes place to restore proper alignment and generally support vertebral members. During surgical treatment, one or more rods and bone fasteners can be delivered to a surgical site. The rods may be attached via the fasteners to the exterior of two or more vertebral members.

Examples of prior art are given, e.g., in US 2016/0166289 A1, which generally discloses a rod connector for adding a pedicle rod to an existing spinal fusion construct, and which specifically discloses an open rod connector that has a swiveling rod collet that allows for a pedicle rod to be rotated into the desired alignment.

This disclosure describes an improvement over these prior technologies.

### SUMMARY

The present invention provides a spinal construct according to independent claim 1. Further embodiments are described in the dependent claims. Associated surgical methods are also described herein to aid understanding the invention. These methods do not form part of the claimed invention.

### BRIEF DESCRIPTION OF THE DRAWINGS

FIGS. 1-4 as such do not show embodiments of the claimed invention, but may help understanding the invention. The present disclosure will become more readily apparent from the specific description accompanied by the following drawings, in which:
FIG. 1 is a perspective view of components of one example of a system in accordance with the principles of the present disclosure;
FIG. 2 is a plan view of the components shown in FIG. 1;
FIG. 3 is a side view of the components shown in FIG. 1;
FIG. 4 is a perspective view of components of one example of a system in accordance with the principles of the present disclosure disposed with vertebrae;
FIG. 5 is a perspective view of components of one embodiment of a system in accordance with the principles of the present disclosure;
FIG. 6 is a side view of the components shown in FIG. 5;
FIG. 7 is a plan view of the components shown in FIG. 5;
FIG. 8 is a side view of the components shown in FIG. 5; and
FIG. 9 is a perspective view of components of one embodiment of a system in accordance with the principles of the present disclosure disposed with vertebrae.

### DETAILED DESCRIPTION

The exemplary embodiments of the surgical system disclosed are discussed in terms of medical devices for the treatment of musculoskeletal disorders and more particularly, in terms of a surgical system.

In some embodiments, the systems of the present disclosure are employed with a spinal joint fusion, for example, with a cervical, thoracic, lumbar and/or sacral region of a spine.

In some embodiments, the present surgical system includes a spinal construct comprising a connector. In some embodiments, the spinal construct includes one rod slot, such as, for example, a passageway configured for attachment with a spinal implant, such as, for example, a spinal rod mounted to vertebrae by, such as, for example, pedicle screws and/or hooks. In some embodiments, the spinal construct includes a second passageway configured for attachment with a spinal implant, such as, for example, a spinal rod to extend an existing construct to one or more adjacent levels. In some embodiments, the spinal construct includes a connector having a side loading passageway and a top loading passageway.

In some embodiments, the spinal construct includes a sagittal adjusting saddle disposed in a top loading passageway. In some embodiments, the sagittal adjusting saddle is configured to accommodate various rod angulations in a sagittal plane of vertebrae. In some embodiments, the sagittal adjusting saddle is configured to improve strength of the spinal construct. In some embodiments, the spinal construct includes a saddle in at least one of the passageways to facilitate sagittal adjustment of a rod while the first and second passageways are fixed to each other.

In some embodiments, one or all of the components of the surgical system may be disposable, peel-pack, pre-packed sterile devices. One or all of the components of the system may be reusable. The system may be configured as a kit with multiple sized and configured components.

In some embodiments, the surgical system of the present disclosure may be employed to treat spinal disorders such as, for example, degenerative disc disease, disc herniation, osteoporosis, spondylolisthesis, stenosis, scoliosis and other curvature abnormalities, kyphosis, tumor and fractures. In some embodiments, the surgical system of the present disclosure may be employed with other osteal and bone related applications, including those associated with diagnostics and therapeutics. In some embodiments, the disclosed surgical system may be alternatively employed in a surgical treatment with a patient in a prone or supine position, and/or employ various surgical approaches to the spine, including anterior, posterior, posterior mid-line, direct lateral, postero-lateral, and/or antero-lateral approaches, and in other body regions. The surgical system of the present disclosure may also be alternatively employed with procedures for treating the lumbar, cervical, thoracic, sacral and pelvic regions of a spinal column. The surgical system of the present disclosure may also be used on animals, bone models and other non-living substrates, such as, for example, in training, testing and demonstration.

The surgical system of the present disclosure may be understood more readily by reference to the following detailed description of the embodiments taken in connection with the accompanying drawing figures, which form a part of this disclosure. t is to be understood that this application is not limited to the specific devices, methods, conditions or parameters described and/or shown herein, and that the terminology used herein is for the purpose of describing particular embodiments by way of example only and is not intended to be limiting. In some embodiments, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it wi II be understood that the particular value forms another embodiment. It is also understood that all spatial references, such as, for example, horizontal, vertical, top, upper, lower, bottom, left and right, are for illustrative purposes only and can be varied within the scope of the disclosure. For example, the references "upper" and "lower" are relative and used only in the context to the other, and are not necessarily "superior" and "inferior".

As used in the specification and including the appended claims, "treating" or "treatment" of a disease or condition refers to performing a procedure that may include administering one or more drugs to a patient (human, normal or otherwi se or other mammal), employing implantable devices, and/or employing instruments that treat the disease, such as, for example, microdiscectomy instruments used to remove portions bulging or herniated discs and/or bone spurs, in an effort to alleviate signs or symptoms of the disease or condition. Alleviation can occur prior to signs or symptoms of the disease or condition appearing, as well as after their appearance. Thus, treating or treatment includes preventing or prevention of disease or undesirable condition (e.g., preventing the disease from occurring in a patient, who may be predisposed to the disease but has not yet been diagnosed as having it). In addition, treating or treatment does not require complete alleviation of signs or symptoms, does not require a cure, and specifically includes procedures that have only a marginal effect on the patient. Treatment can include inhibiting the disease, e.g., arresting its development, or relieving the disease, e.g., causing regression of the disease. For example, treatment can include reducing acute or chronic inflammation; alleviating pain and mitigating and inducing regrowth of new ligament, bone and other tissues; as an adjunct in surgery; and/or any repair procedure. In some embodiments, as used in the specification and including the appended claims, the term "tissue" includes soft tissue, ligaments, tendons, cartilage and/or bone unless specifically referred to otherwise.

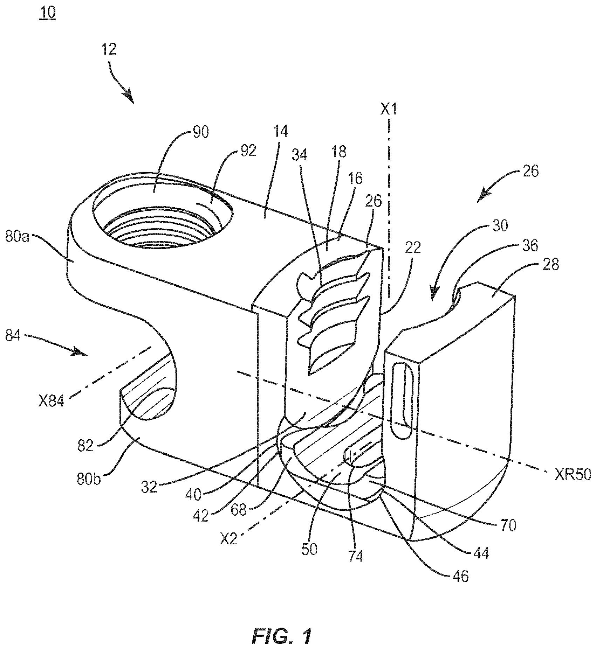

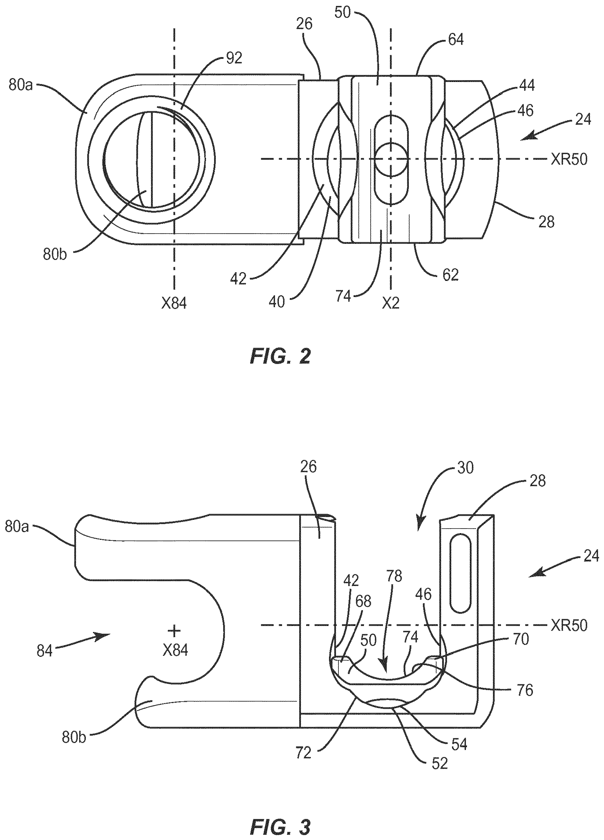

The following discussion includes a description of a surgical system including a spinal construct, related components and methods of employing the surgical system in accordance with the principles of the present disclosure. Alternate embodiments are also disclosed. Reference is made in detail to the exemplary embodiments of the present disclosure, which are illustrated in the accompanying figures. Turning to FIGS. 1-3, there are illustrated components of a surgical system, such as, for example, a spinal implant system 10.

The components of spinal implant system 10 can be fabricated from biologically acceptable materials suitable for medical applications, including metals, synthetic polymers, ceramics and bone material and/or their composites. For example, the components of spinal implant system 10, individually or collectively, can be fabricated from materials such as stainless steel alloys, aluminum, commercially pure titanium, titanium alloys, Grade 5 titanium, super-elastic titanium alloys, cobalt-chrome alloys, superelastic metallic alloys (e.g., Nitinol, super elasto-plastic metals, such as GUM METAL^{®}), ceramics and composites thereof such as calcium phosphate (e.g., SKELITE^{™}), thermoplastics such as polyaryletherketone (PAEK) including polyetheretherketone (PEEK), polyetherketoneketone (PEKK) and polyetherket one (PEK), carbon-PEEK composites, PEEK-BaS04 polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polyaetide, polyglycolide, polytyrosine carbonate, polycaroplaetohe and their combinations.

Various components of spinal implant system 10 may have material composites, including the above materials, to achieve various desired characteristics such as strength, rigidity, elasticity, compliance, biomechanical performance, durability and radiolucency or imaging preference. The components of spinal implant system 10, individually or collectively, may also be fabricated from a heterogeneous material such as a combination of two or more of the above-described materials. The components of spinal implant system 10 may be monolithically formed, integrally connected or include fastening elements and/or instruments, as described herein.

Spinal implant system 10 comprises a spinal construct, such as, for, example, a connector 12. In some embodiments, connector 12 is configured to accommodate various angles of a spinal implant in a plane of a body, such as, for example, transverse, vertical, horizontal, diagonal, coronal and/or sagittal planes of vertebrae.

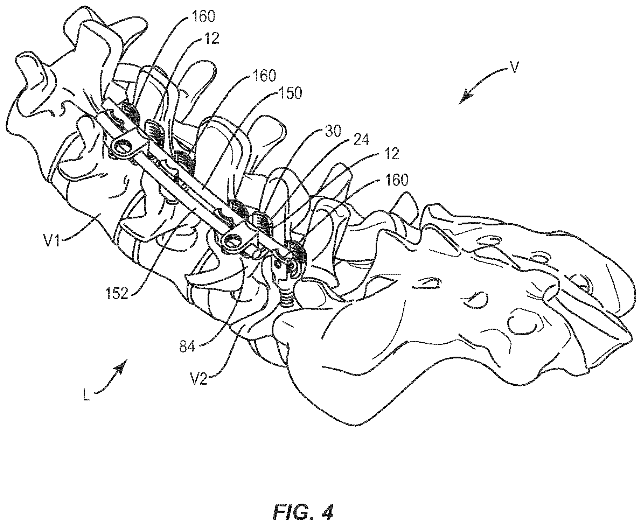

Connector 12 includes a body 14 having a surface 16 that defines a wall 18. Wall 18 includes a surface 22 that defines a portion of a cavity, such as for example, a receiver 24. Receiver 24 includes a pair of spaced apart arms 26, 28 that define an implant cavity, such as, for example, a passageway 30 therebetween. Passageway 30 is configured for top loading of a spinal implant, such as, for example, a spinal rod 150, as shown in FIG. 4. Arms 26, 28 each extend parallel to an axis X1, as shown in FIG. 1. In some embodiments, arm 26 and/or arm 28 may be disposed at alternate orientations, relative to axis X1, such as, for example, transverse, perpendicular and/or other angular orientations such as acute or obtuse, coaxial and/or may be offset or staggered. Arms 26, 28 each include an arcuate outer surface extending between a pair of side surfaces. In some embodiments, at least one of the outer surfaces and the side surfaces of arms 26, 28 have at least one recess or cavity therein configured to receive an insertion tool, compression instrument and/or instruments for manipulating connector 12.

Passageway 30 is substantially U-shaped. In some embodiments, all or only a portion of passageway 30 may have alternate cross section configurations, such as, for example, closed, V-shaped, W-shaped, oval, oblong triangular, square, polygonal, irregular, uniform, non-uniform, offset, staggered, and/or tapered. Receiver 24 includes an inner surface 32. A portion of surface 32 includes a thread form 34 located adjacent arm 26 and a thread form 36 located adjacent arm 28. Thread forms 34, 36 are each configured for engagement with a coupling member, such as, for example, a set screw (not shown), to retain a spinal rod within passageway 30. In some embodiments, surface 32 may be disposed with a set screw in alternate fixation configurations, such as, for example, friction fit, pressure fit, locking protrusion/recess, locking keyway and/or adhesive. In some embodiments, all or only a portion of surface 32 may have alternate surface configurations to enhance engagement with a spinal rod and/or a set screw such as, for example, rough, arcuate, undulating, mesh, porous, semi -porous, dimpled and/or textured.

The set screw is configured for engagement with a spinal rod to facilitate fixation and/or locking of the spinal rod with receiver 24. A set screw is disposable with receiver 24 between a non-locking orientation, such that a spinal rod is translatable relative to connector 12 and a locked orientation, such that a set screw fixes the spinal rod with connector 12.

Arms 26, 28 are configured to support relative movement of a part, such as, for example, a saddle 50, as described herein. Arm 26 includes a surface 40 that defines a track 42 adjacent arm 26. Arm 28 includes a surface 44 that defines a track 46 adjacent arm 28. Tracks 42, 46 are configured to facilitate translation of saddle 50 relative to receiver 24, as described herein. Arms 26, 28 are configured to guide saddle 50 along tracks 42, 46 relative to receiver 24. Receiver 24 includes a surface 52 that defines an arcuate portion 54 configured for disposal of at least a portion of a spinal rod, which may be positioned with receiver 24.

Saddle 50 extends between an end 62 and an end 64. Saddle 50 includes a surface 66 defining a wall 68 and a wall 70. Walls 68, 70 are configured to fit within the outer profile and/or perimeter of receiver 24. In some embodiments, saddle 50 fits within the outer profile and/or perimeter of receiver 24. Saddle 50 includes a surface 72 configured for slidable engagement with tracks 42, 46. Surface 72 extends between ends 62, 64 and is configured for slidable engagement with receiver 24 along an arcuate pathway of the components. Saddle 50 includes a surface 74 configured to engage at least a portion of a spinal rod and is moveable relative to receiver 24 in a plane, such as, for example, a sagittal plane of a body and/or vertebrae. Surface 74 defines a concave surface 76 that defines an implant cavity 78. Passageway 30 includes cavity 78.

Receiver 24 defines an axis X2 oriented transverse to axis X1. Saddle 50 is configured to receive and movably support a spinal rod such that the spinal rod can translate axially, rotate and/or pivot relative to receiver 24 along and about axis X2 prior to fixation with saddle 50. In some embodiments, a spinal rod may be disposed within passageway 30 for relative movement in orientations relative to axis X2, such as, for example, transverse, perpendicular and/or other angular orientations such as acute or obtuse, co-axial and/or may be offset or staggered. In some embodiments, axis X2 may be disposed at angular orientations relative to axis X1, such as, for example, acute or obtuse.

In some embodiments, saddle 50 may be elastic and pliable in a configuration to react to forces applied and/or force changes, such as, for example, positioning treatment, patient growth, trauma and degeneration, and/or component creep, deformation, damage and degeneration, to maintain the applied force transmitted from an implant positioned in passageway 30 substantially constant. In some embodiments, saddle 50 can facilitate maintenance of a holding force on a spinal rod positioned in passageway 30 to remain the holding force relatively constant despite growth and changes.

Saddle 50 translates relative to receiver 24 via relative slidable translation along tracks 42, 46 such that saddle 50 is rotatable relative to receiver 24 in a plane, such as, for example, a sagittal plane of a body and/or vertebrae. Saddle 50 is rotatable about an axis XR50 through an angular range. Saddle 50 is pivotable along the arcuate path of receiver 24 through passageway 30 relative to axis X 1. In some embodiments, the angular range may include a range of approximately +/- 0 to 30 degrees and/or 0 to 30 degrees in opposing directions. In some embodiments, saddle 50 is disposed with receiver 24 for relative movement of receiver 24 in orientations relative to axis XR50, such as, for example, transverse, perpendicular and/or other angular orientations such as acute or obtuse, co-axial and/or may be offset or staggered. In some embodiments, saddle 50 moves relative to receiver 24 in alternate planes relative to a body, such as, for example, vertical, horizontal, diagonal, transverse, coronal and/or sagittal planes of a body. In some embodiments, axis XR50 may be disposed at alternate orientations, relative to axis X2, such as, for example, perpendicular, transverse, parallel and/or other angular orientations such as acute or obtuse, coaxial and/or may be offset or staggered.

Wall 18 includes extensions 80a, 80b. Extensions 80a, 80b are disposed in a spaced apart relation. Extensions 80a, 80b extend perpendicular to axis X1. In some embodiments, extension 80a and/or extension 80b may be disposed at alternate orientations, relative to axis X1, such as, for example, transverse and/or other angular orientations such as acute or obtuse, coaxial and/or may be offset or staggered. Extensions 80a, 80b and a surface 82 of wall 18 define an opening, such as, for example, a passageway 84. Passageway 84 is configured for disposal of a longitudinal element, such as, for example, a spinal rod 152, as shown in FIG. 4. Passageway 84 is configured for side loading of spinal rod 152, as described herein. Passageway 84 extends along an axis X84. In some embodiments, axis X84 may be disposed at alternate orientations relative to axis X2, such as, for example, parallel, transverse, perpendicular and/or other angular orientations such as acute or obtuse, coaxial and/or may be offset or staggered. In some embodiments, axis X84 may be offset relative to axis X2, for example, above, below, posterior and/or anterior. In some embodiments, spinal rod 152 may be disposed at alternate orientations relative to spinal rod 150, such as, for example, parallel , transverse, perpendicular and/or other angular orientations such as acute or obtuse, coaxial and/or may be offset or staggered. In some embodiments, spinal rod 152 may be offset relative to spinal rod 150, for example, above, below, posterior and/or anterior.

In some embodiments, passageway 84 is disposed separate and apart from passageway 30. In some embodiments, passageway 84 is disposed in a side by side orientation relative to passageway 30. In some embodiments, passageway 84 is disposed in a parallel orientation relative to passageway 30. In some embodiments, passageway 84 is disposed transverse to passageway 30. In some embodiments, passageway 84 may be disposed in various orientations, such as, for example, perpendicular, transverse and/or at angular orientations, such as acute or obtuse relative to passageway 30. In some embodiments, passageway 84 may be disposed offset or staggered from passageway 30. In some embodiments, passageway 84 may have various cross section configurations, such as, for example, oval, oblong, triangular, rectangular, square, polygonal, irregular, uniform, non-uniform, variable and/or tapered. In some embodiments, surface 82 may include gripping elements or surfaces, such as, for example, rough, arcuate, undulating, mesh, porous, semi-porous, dimpled and/or textured to facilitate engagement with a spinal rod.

Body 14 includes a surface 90 that defines a cavity, such as, for example, an opening 92. Surface 90 is threaded and configured for disposal of a coupling member, such as, for example, a set screw (not shown). The set screw is configured for engagement with a spinal rod to facilitate fixation and/or locking of the spinal rod with connector 12. The set screw is disposable between a non-locking orientation, such that the spinal rod is translatable relative to connector 12 and a locked orientation, such that the set screw fixes the spinal rod with connector 12.

In some embodiments, a portion of connector 12 is attachable with a shaft configured to penetrate tissue, such as, for example, bone. In some embodiments, the shaft includes a threaded surface to facilitate engagement with tissue. In some embodiments, receiver 24 is attachable with the shaft to form a bone screw. In some embodiments, connector 12 is monolithically formed, integrally connected or includes fastening elements and/or instruments for connection with a bone screw shaft. In some embodiments, connector 12 includes a bone screw shaft and comprises, for example, multi-axial screws, sagittal angulation screws, pedicle screws, mono-axial screws, uni-planar screws, facet screws, fixed screws, tissue penetrating screws, conventional screws, expanding screws, wedges, anchors, buttons, clips, snaps, friction fittings, compressive fittings, expanding rivets, staples, nails, adhesives, posts, fixation plates and/or posts.

In some embodiments, spinal implant system 10 can include one or a plurality of connectors 12 such as those described herein and/or fixation elements, which may be employed with a single vertebral level or a plurality of vertebral levels. In some embodiments, connectors 12 may be engaged with vertebrae in various orientations, such as, for example, series, parallel, offset, staggered and/or alternate vertebral levels. In some embodiments, connectors 12 may be configured as multi-axia I screws, sagittal angulation screws, pedicle screws, mono-axial screws, uni-planar screws, fixed screws, anchors, tissue penetrating screws, conventional screws, expanding screws. In some embodiments, connectors 12 may be employed with wedges, anchors, buttons, clips, snaps, friction fittings, compressive fittings, expanding rivets, staples, nails, adhesives, posts, connectors, fixation plates and/or posts.

In assembly, operation and use, spinal implant system 10, similar to the systems and methods described herein, is employed with a surgical procedure, such as, for example, a correction treatment of an applicable condition or injury of an affected section of a spinal column and adjacent areas within a body. Spinal implant system 10 may be completely or partially revised, removed or replaced.

In use, to treat a selected section of vertebrae V, including vertebrae V1, V2, as shown in FIG. 4, a medical practitioner obtains access to a surgical site including vertebrae V in any appropriate manner, such as through incision and retraction of tissues. In some embodiments, spinal implant system 10 can be used in any existing surgical method or technique including open surgery, mini-open surgery, minimally invasive surgery and percutaneous surgical implantation, whereby vertebrae V is accessed through a mini-incision, or a sleeve that provides a protected passageway to the area. Once access to the surgical site is obtained, the particular surgical procedure can be performed for treating the spine disorder.

An incision is made in the body of a patient and a cutting instrument (not shown) creates a surgical pathway for implantation of components of spinal implant system 10. A preparation instrument (not shown) can be employed to prepare tissue surfaces of vertebrae V, as well as for aspiration and irrigation of a surgical region.

Bone screws 160 are engaged with vertebrae V along a lateral side L of vertebrae V, as shown in FIG. 4. Bone screws 160 are manipulated to drive, torque, insert or otherwise connect bone screws 160 with vertebrae V. Spinal rod 150 is delivered along the surgical pathway to a surgical site adjacent vertebrae V. Spinal rod 150 is disposed with bone screws 160 along vertebrae V. Connectors 12 are disposed adjacent spinal rod 150. Each connector 12 is manipulated to dispose spinal rod 150 with passageway 30 and saddle 50 from a top loading orientation.

Saddle 50 receives and movably supports spinal rod 150 such that spinal rod 150 is movable within passageway 30. Tracks 42, 46 facilitate translation of saddle 50 relative to receiver 24. In some embodiments, saddle 50 is selectively translatable along tracks 42, 46 and the arcuate path of saddle 50 relative to receiver 24 in the sagittal plane to accommodate sagittal anatomical differences. Connector 12 provides angular accommodation in a sagittal plane of vertebrae V.

Spinal rod 150 is fixed with receiver 24 with a set screw (not shown). The set screw is engaged with a surgical instrument, such as, for example, a driver (not shown), which advances the set screw into engagement with arms 26, 28 in a locking orientation, as described herein. The driver engages the set screw to fix spinal rod 150 with receiver 24 and for attachment of spinal rod 150 with vertebrae V.

Spinal rod 152 is delivered along the surgical pathway to a surgical site adjacent vertebrae V. Spinal rod 152 is disposed with passageway 84 from a side loading orientation. A set screw (not shown) is engaged with opening 92. The set screw is engaged with a surgical instrument, such as, for example, a driver (not shown), which advances the set screw into opening 92 in a non-locking orientation, as described herein. Spinal rod 152 is translatable relative to connector 12 to position spinal rod 152 relative to spinal rod 150 and connector 12. The driver engages the set screw to fix spinal rod 152 with connector 12 and for attachment of spinal rod 152 with vertebrae V. In some embodiments, spinal rod 152 is configured to share the load applied to spinal rod 150. In some embodiments, spinal rod 152 is configured to extend spinal rod 150 to an adjacent vertebral level. Spinal rod 152 is configured to add support and strength to spinal implant system 10 along vertebrae V. In some embodiments, one or more connectors 12 of spinal implant system 10, as described herein, for example, as shown in FIG. 4, can be oriented such that spinal rod 150 is disposed with passageway 84, and/or spinal rod 152 is disposed with passageway 30 and supported by saddle 50, similar to that described herein.

In some embodiments, spinal implant system 10 includes a second set of connectors 12, bone screws 160 and spinal rods 150, 152 (not shown) delivered along the surgical pathway to the surgical site adjacent a contra-lateral side of vertebrae V. The second set of connectors 12, bone screws 160 and spinal rods 150, 152 are connected with the contra-lateral side of vertebrae V, similar to lateral side L described herein. In some embodiments, the spinal constructs of spinal implant system 10, as described herein, are fixed with vertebrae V in a side by side orientation and/or a bi-lateral arrangement to stabilize vertebrae V and affect growth for a correction treatment to treat spine pathologies, as described herein. In some embodiments, one or all of the components of spinal implant system 10 can be delivered or implanted as a preassembled device or can be assembled in situ, in a selected order of assembly or the order of assembly of the particular components of system 10 can be varied according to practitioner preference, patient anatomy or surgical procedure parameters.

Upon completion of the procedure, the surgical instruments, assemblies and non-implanted components of spinal implant system 10 are removed from the surgical site and the incision is closed. One or more of the components of spinal implant system 10 can be made of radiolucent materials such as polymers. Radiomarkers may be included for identification under x-ray, fluoroscopy, CT or other imaging techniques. In some embodiments, the use of surgical navigation, microsurgical and image guided technologies may be employed to access, view and repair spinal deterioration or damage, with the aid of spinal implant system 10.

h some embodiments, spinal implant system 10 includes an agent, which may be disposed, packed, coated or layered within, on or about the components and/or surfaces of spinal implant system 10. In some embodiments, the agent may include bone growth promoting material, such as, for example, bone graft to enhance fixation of the bone fasteners with vertebrae. In some embodiments, the agent may include one or a plurality of therapeutic agents and/or pharmacological agents for release, including sustained release, to treat, for example, pain, inflammation and degeneration.

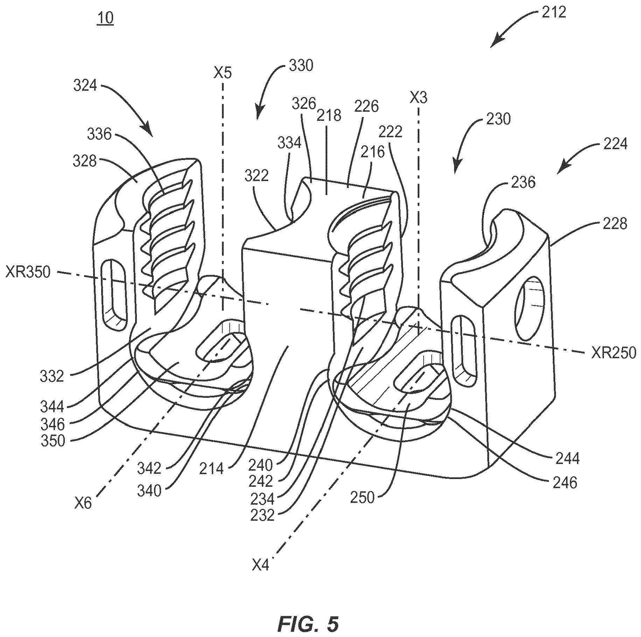

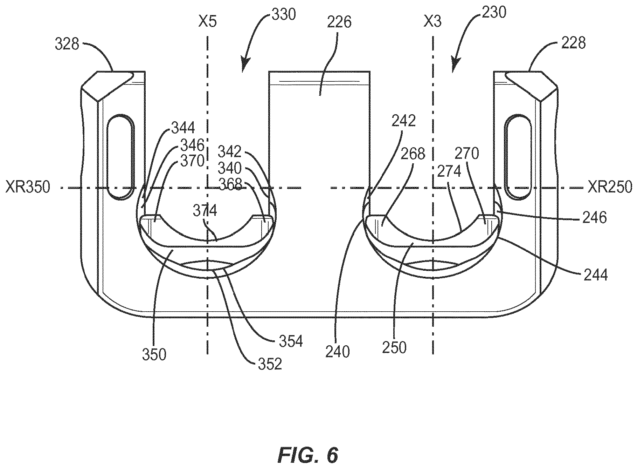

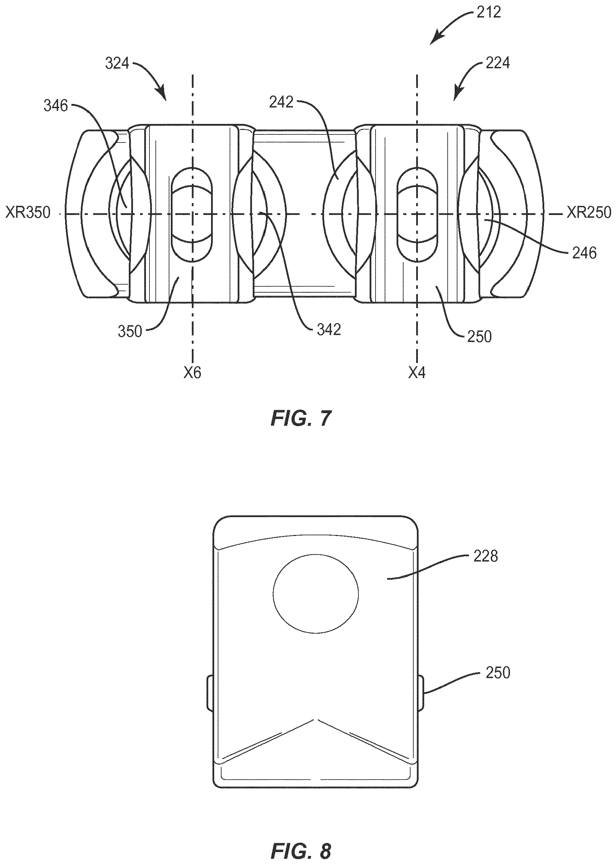

In one embodiment, as shown in FIGS. 5-8, spinal implant system 10 includes a connector 212, similar to connector 12 described herein. Connector 212 is configured to accommodate various angles of a spinal implant in a plane of a body, such as, for example, transverse, vertical, horizontal, diagonal, coronal and/or sagittal planes of vertebrae.

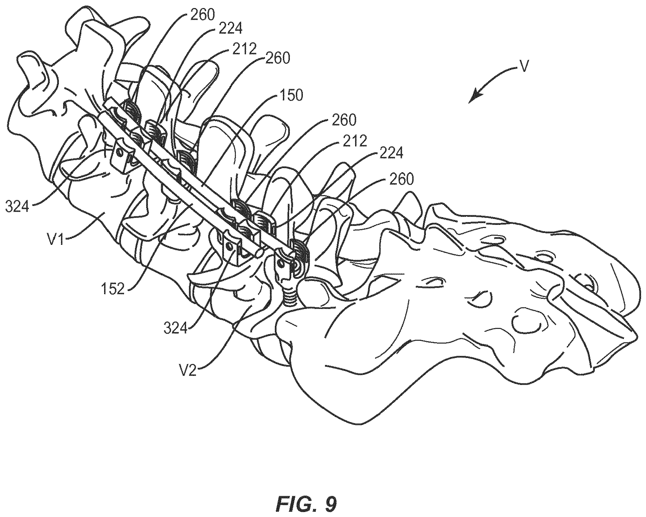

Connector 212 includes a body 214 having a surface 216 that defines a wall 218. Wall 218 includes a surface 222 that defines a portion of a cavity, such as for example, a receiver 224, similar to receiver 24 described herein. Receiver 224 includes a pair of spaced apart arms 226, 228 that define a passageway 230 therebetween Passageway 230 is configured for top loading of a spinal rod 150, as shown in FIG. 9. Arms 226, 228 each extend parallel to an axis X3, as shown in FIG. 5.

Passageway 230 is substantially U-shaped. Receiver 224 includes an inner surface 232. A portion of surface 232 includes a thread form 234 located adjacent arm 226 and a thread form 236 located adjacent arm 228. Thread forms 234, 236 are each configured for engagement with a coupling member, such as, for example, a set screw (not shown), to retain a spinal rod within passageway 230.

Arms 226, 228 are configured to support relative movement of a saddle 250, similar to saddle 50 described herein. Arm 226 includes a surface 240 that defines a track 242 adjacent arm 226. Arm 228 includes a surface 244 that defines a track 246 adjacent arm 228. Tracks 242, 246 are configured to facilitate translation of saddle 250 relative to receiver 224, as described herein. Arms 226, 228 are configured to guide saddle 250 along tracks 242, 246 relative to receiver 224. Receiver 224 includes a surface 252 that defines an arcuate portion 254 configured for disposal of at least a portion of a spinal rod, which may be positioned with receiver 224.

Saddle 250 includes a wall 268 and a wall 270. Walls 268, 270 are configured to fit within the outer profile and/or perimeter of receiver 224. Saddle 250 is configured for slidable engagement with tracks 242, 246. Saddle 250 includes a surface 274 configured to engage at least a portion of a spinal rod and is moveable relative to receiver 224 in a plane, such as, for example, a sagittal plane of a body and/or vertebrae.

Receiver 224 defines an axis X4 oriented transverse to axis X3. Saddle 250 is configured to receive and movably support a spinal rod such that the spinal rod can translate axially, rotate and/or pivot relative to receiver 224 along and about an axis XR250 prior to fixation with saddle 250.

Saddle 250 translates relative to receiver 224 via relative slidable translation along tracks 242, 246 such that saddle 250 is rotatable relative to receiver 224 in a plane, such as, for example, a sagittal plane of a body and/or vertebrae. Saddle 250 is rotatable about axis XR250 through an angular range, as described herein. Saddle 250 is pivotable along the arcuate path of receiver 224 through passageway 230 relative to axis X3.

Wall 218 includes a surface 322 that defines a portion of a cavity, such as for example, a receiver 324, similar to receiver 24 described herein. Receiver 324 includes arm 226 and an arm 328 spaced apart to define a passageway 330 therebetween. Passageway 330 is configured for top loading of spinal rod 152, as shown in FIG. 9. Arms 326, 328 each extend parallel to an axis XS, as shown in FIG. 5. In some embodiments, receivers 224, 324 form an w-shaped cross section configuration of connector 212.

Passageway 330 is substantially U-shaped. Receiver 324 includes an inner surface 332. A portion of surface 332 includes a thread form 334 located adjacent arm 326 and a thread form 336 located adjacent arm 328. Thread forms 334, 336 are each configured for engagement with a coupling member, such as, for example, a set screw (not shown), to retain a spinal rod within passageway 330.

Arms 326, 328 are configured to support relative movement of a saddle 350, similar to saddle 50 described herein. Arm 326 includes a surface 340 that defines a track 342 adjacent arm 326. Arm 328 includes a surface 344 that defines a track 346 adjacent arm 328. Tracks 342, 346 are configured to facilitate translation of saddle 350 relative to receiver 324, as described herein. Arms 326, 328 are configured to guide saddle 350 along tracks 342, 346 relative to receiver 324. Receiver 324 includes a surface 352 that defines an arcuate portion 354 configured for disposal of at least a portion of a spinal rod, which may be positioned with receiver 324.

Saddle 350 includes a wall 368 and a wall 370. Walls 368, 370 are configured to fit within the outer profile and/or perimeter of receiver 324. Saddle 350 is configured for slidable engagement with tracks 342, 346. Saddle 350 includes a surface 374 configured to engage at least a portion of a spinal rod and is moveable relative to receiver 324 in a plane, such as, for example, a sagittal plane of a body and/or vertebrae.

Receiver 324 defines an axis X6 oriented transverse to axis X5. Axis X6 is orientated parallel to axis X4, as shown in FIG. 5. Saddle 350 is configured to receive and movably support a spinal rod such that the spinal rod can translate axially, rotate and/or pivot relative to receiver 324 along and about axis X6 prior to fixation with saddle 350. Saddle 350 translates relative to receiver 324 via relative slidable translati on along tracks 342, 346 such that saddle 350 is rotatable relative to receiver 324 in a plane, such as, for example, a sagittal plane of a body and/or vertebrae. Saddle 350 is rotatable about an axis XR350 through an angular range, as described herein. Saddle 350 is pivotable along the arcuate path receiver 324 through passageway 330 relative to axis XR350. In some embodiments, connector 212 may include a bone screw shaft, similar to connector 12 described herein. In some embodiments, axis XR350 may be disposed at alternate orientations, relative to axis X R250, such as, for example, coaxial, parallel, transverse, perpendicular and/or other angular orientations such as acute or obtuse, coaxial and/or may be offset or staggered. In some embodiments, axis XR350 may be offset relative to axis XR250, for example, above, below, posterior and/or anterior, such that spinal rod 152 may be offset relative to spinal rod 150, for example, above, below, posterior and/or anterior. In some embodiments, such an offset configuration of spinal rod 152 relative to spinal rod 150 allows disposal of rods 150, 152 in closer proximity for connection with tissue. In some embodiments, such an offset configuration of spinal rod 152 relative to spinal rod 150 allows disposal of rods 150, 152 in an orientation with tissue for avoidance and/or clearance of selected bony anatomy and tissue.

In assembly, operation and use, spinal implant system 10 including connector 212 is employed with a surgical procedure, such as, for example, a correction treatment of an applicable condition or injury of an affected section of a spinal column and adjacent areas within a body. To treat a selected section of vertebrae V, including vertebrae V1, V2, as shown in FIG. 9, a medical practitioner obtains access to a surgical site including vertebrae V, similar to that described herein.

Bone screws 260 are engaged with vertebrae V along a lateral side L of vertebrae V, as shown in FIG. 9. Bone screws 260 are manipulated to drive, torque, insert or otherwise connect bone screws 260 with vertebrae. Spinal rod 150 is delivered along the surgical pathway to a surgical site adjacent vertebrae V. Spinal rod 150 is disposed with bone screws 260 along vertebrae V. Connectors 212 are disposed adjacent spinal rod 150. Spinal rod 150 is movable for disposal with passageway 230 and saddle 250 from a top loading orientation.

Saddle 250 receives and movably supports spinal rod 150 such that spinal rod 150 is movable within passageway 230. Tracks 242, 246 facilitate translation of saddle 250 relative to receiver 224. In some embodiments, saddle 250 is selectively translatable along tracks 242, 246 and the arcuate path of saddle 250 relative to receiver 224 in the sagittal plane to accommodate sagittal anatomical differences. Connector 212 provides angular accommodation in a sagittal plane of vertebrae V.

Spinal rod 150 is fixed with receiver 224 with a set screw (not shown). The set screw is engaged with a surgical instrument, such as, for example, a driver (not shown), which advances the set screw into engagement with arms 226, 228 in a locking orientation, as described herein. The driver engages the set screw to fix spinal rod 150 with receiver 224 and for attachment of spinal rod 150 with vertebrae V.

Spinal rod 152 is delivered along the surgical pathway to a surgical site adjacent vertebrae V. Spinal rod 152 is disposed with saddle 350, which receives and movably supports spinal rod 152 such that spinal rod 152 is movable within passageway 330. Tracks 342, 346 facilitate translation of saddle 350 relative to receiver 324. In some embodiments, saddle 350 is selectively translatable along tracks 342, 346 and the arcuate path of saddle 350 relative to receiver 324 in the sagittal plane to accommodate sagittal anatomical differences. Connector 212 provides angular accommodation in a sagittal plane of vertebrae V.

Spinal rod 152 is fixed with receiver 324 with a set screw (not shown). The set screw is engaged with a surgical instrument, such as, for example, a driver (not shown), which advances the set screw into engagement with arms 326, 328 in a locking orientation, as described herein. The driver engages the set screw to fix spinal rod 152 with receiver 324 and for attachment of spinal rod 152 with vertebrae V. In some embodiments, spinal rod 152 is configured to share the load applied to spinal rod 150. In some embodiments, spinal implant system 10 includes a second set of connectors 212, bone screws 260 and spinal rods 150, 152 (not shown) delivered along the surgical pathway to the surgical site adjacent a contra-lateral side of vertebrae V. The second set of connectors 212, bone screws 260 and spinal rods 150, 152 are connected with the contra-lateral side of vertebrae V, similar to lateral side L described herein.

## Claims

1. A spinal construct (212), comprising:
a body (214) connectable with tissue and including a wall (218) disposed between a first implant cavity (230) and a second implant cavity (330),
wherein the first implant cavity (230) includes a top loading passageway (230) defined by a pair of spaced apart arms (226, 228) and the second implant cavity (330) includes another top loading passageway (330) defined by another pair of spaced apart arms (326, 328),
wherein the arms (226, 228) of the pair of spaced apart arms (226, 228) each extend parallel to a first axis (X3) and each include a surface (240, 244) that defines a track (242, 246) adjacent thereto,
wherein the arms (326, 328) of the other pair of spaced apart arms (326, 328) each extend parallel to a second axis (X5) and each include a surface (340, 344) that defines a track (342, 346) adjacent thereto, and
a part (250) disposed with the top loading passageway (230) and another part (350) disposed with the other top loading passageway (330), each of the parts (250, 350) being slidable in a translatory manner along the tracks (242, 246), and a corresponding arcuate path, adjacent the pair of spaced apart arms (226, 228) and the tracks (342, 346), and a corresponding arcuate path, adjacent the other pair of spaced apart arms (326, 328), respectively, prior to fixation, relative to the wall (218) so that the part (250) is rotatable about a third axis (XR250), which extends transverse to the first axis (X3), and that the other part (350) is rotatable about a fourth axis (XR350), which extends transverse to the second axis (X5),
wherein the part (250) and the other part (350) are pivotable relative to the wall (218) relative to the first axis (X3) and to the second axis (X5), respectively.

2. A spinal construct (212) as recited in Claim 1, wherein the cavities (230, 330) are disposed in a side by side orientation.

3. A spinal construct (212) as recited in Claim 1 or 2, wherein the wall (218) includes a first surface (222) that defines at least a portion (224) of the first cavity and a second surface (322) that defines at least a portion (324) of the second cavity.

4. A spinal construct (212) as recited in one of Claims 1-3, wherein the first cavity (230) is adjacent to and spaced apart from the second cavity (330).

5. A spinal construct (212) as recited in one of Claims 1-4, wherein the body (214) includes a w-shaped cross section configuration.

6. A spinal construct (212) as recited in one of Claims 1-5, wherein the body (214) includes a connector.

7. A spinal construct (212) as recited in one of Claims 1-6, wherein the body (214) includes an implant receiver of a bone fastener having a shaft configured to penetrate tissue, the implant receiver configured for disposal of at least one spinal rod.

8. A spinal construct (212) as recited in one of Claims 1-7, wherein the cavities (230, 330) are disposed in a relatively parallel orientation.

9. A spinal construct (212) as recited in one of Claims 1-8, further comprising a first spinal rod (150) disposed in the first cavity (230) and a second spinal rod (152) disposed in the second cavity (330).

10. A spinal construct (212) as recited in one of Claims 1-9, wherein the parts (250, 350) are pivotable in a range of approximately +/- 0 to 30 degrees relative to the wall (218).

11. A spinal construct (212) as recited in one of Claims 1-10, wherein the part (250, 350) defines a first surface (74) configured for slidable engagement with the body (214) and a second concave surface (76) configured for engagement with a spinal rod.

## Patentansprüche

1. Wirbelsäulenkonstrukt (212), das Folgendes umfasst:
einen Körper (214), der mit Gewebe verbindbar ist und eine Wand (218) beinhaltet, die zwischen einem ersten Implantathohlraum (230) und einem zweiten Implantathohlraum (330) angeordnet ist,
wobei der erste Implantathohlraum (230) einen Toploading-Durchgang (230) beinhaltet, der durch ein Paar voneinander beabstandeter Arme (226, 228) definiert ist, und der zweite Implantathohlraum (330) einen anderen Toploading-Durchgang (330) beinhaltet, der durch ein anderes Paar voneinander beabstandeter Arme (326, 328) definiert ist,
wobei sich die Arme (226, 228) des Paars beabstandeter Arme (226, 228) jeweils parallel zu einer ersten Achse (X3) erstrecken und jeweils eine Oberfläche (240, 244) beinhalten, die eine Spur (242, 246) definiert, die daran angrenzt,
wobei sich die Arme (326, 328) des anderen Paars beabstandeter Arme (326, 328) jeweils parallel zu einer zweiten Achse (X5) erstrecken und jeweils eine Oberfläche (340, 344) beinhalten, die eine Spur (342, 346) definiert, die daran angrenzt, und
ein Teil (250), das mit dem Toploading-Durchgang (230) angeordnet ist, und ein anderes Teil (350), das mit dem anderen Toploading-Durchgang (330) angeordnet ist, wobei jedes der Teile (250, 350) entlang der Spuren (242, 246) und eines entsprechenden bogenförmigen Pfades, angrenzend an das Paar beabstandeter Arme (226, 228) beziehungsweise der Spuren (342, 346) und eines entsprechenden bogenförmigen Pfades, angrenzend an das andere Paar beabstandeter Arme (326, 328) vor einer Fixierung relativ zu der Wand (218) derart translatorisch verschiebbar ist, dass das Teil (250) um eine dritte Achse (XR250) herum drehbar ist, die sich quer zu der ersten Achse (X3) erstreckt, und dass das andere Teil (350) um eine vierte Achse (XR350) herum drehbar ist, die sich quer zu der zweiten Achse (X5) erstreckt,
wobei das Teil (250) und das andere Teil (350) relativ zu der Wand (218) relativ zu der ersten Achse (X3) beziehungsweise zu der zweiten Achse (X5) schwenkbar sind.

2. Wirbelsäulenkonstrukt (212) nach Anspruch 1, wobei die Hohlräume (230, 330) in einer nebeneinander liegenden Ausrichtung angeordnet sind.

3. Wirbelsäulenkonstrukt (212) nach Anspruch 1 oder 2, wobei die Wand (218) eine erste Oberfläche (222), die wenigstens einen Abschnitt (224) des ersten Hohlraums definiert, und eine zweite Oberfläche (322) beinhaltet, die wenigstens einen Abschnitt (324) des zweiten Hohlraums definiert.

4. Wirbelsäulenkonstrukt (212) nach einem der Ansprüche 1-3, wobei der erste Hohlraum (230) an den zweiten Hohlraum (330) angrenzt und von diesem beabstandet ist.

5. Wirbelsäulenkonstrukt (212) nach einem der Ansprüche 1-4, wobei der Körper (214) eine w-förmige Querschnittskonfiguration beinhaltet.

6. Wirbelsäulenkonstrukt (212) nach einem der Ansprüche 1-5, wobei der Körper (214) einen Verbinder beinhaltet.

7. Wirbelsäulenkonstrukt (212) nach einem der Ansprüche 1-6, wobei der Körper (214) eine Implantataufnahme eines Knochenbefestigungselements beinhaltet, die einen Schaft aufweist, der konfiguriert ist, um Gewebe zu durchdringen, wobei die Implantataufnahme für eine Anordnung wenigstens eines Wirbelsäulenstabs konfiguriert ist.

8. Wirbelsäulenkonstrukt (212) nach einem der Ansprüche 1-7, wobei die Hohlräume (230, 330) in einer relativ parallelen Ausrichtung angeordnet sind.

9. Wirbelsäulenkonstrukt (212) nach einem der Ansprüche 1-8, das ferner einen ersten Wirbelsäulenstab (150), der in dem ersten Hohlraum (230) angeordnet ist, und einen zweiten Wirbelsäulenstab (152) umfasst, der in dem zweiten Hohlraum (330) angeordnet ist.

10. Wirbelsäulenkonstrukt (212) nach einem der Ansprüche 1-9, wobei die Teile (250, 350) in einem Bereich von ungefähr +/- 0 bis 30 Grad relativ zu der Wand (218) schwenkbar sind.

11. Wirbelsäulenkonstrukt (212) nach einem der Ansprüche 1-10, wobei das Teil (250, 350) eine erste Oberfläche (74), die für einen verschiebbaren Eingriff mit dem Körper (214) konfiguriert ist, und eine zweite konkave Oberfläche (76) definiert, die für einen Eingriff mit einer Wirbelsäulenstange konfiguriert ist.

## Revendications

1. Construction spinale (212), comprenant :
un corps (214) pouvant être relié au tissu et comportant une paroi (218) disposée entre une première cavité d'implant (230) et une seconde cavité d'implant (330),
la première cavité d'implant (230) comportant un passage de chargement supérieur (230) défini par une paire de bras espacés (226, 228) et la seconde cavité d'implant (330) comportant un autre passage de chargement supérieur (330) défini par une autre paire de bras espacés (326, 328),
les bras (226, 228) de la paire de bras espacés (226, 228) s'étendant chacun parallèlement à un premier axe (X3) et comportant chacun une surface (240, 244) qui définit une piste (242, 246) à proximité de ceux-ci,
les bras (326, 328) de l'autre paire de bras espacés (326, 328) s'étendant chacun parallèlement à un deuxième axe (X5) et comportant chacun une surface (340, 344) qui définit une piste (342, 346) à proximité de ceux-ci, et
une partie (250) disposée avec le passage de chargement supérieur (230) et une autre partie (350) disposée avec l'autre passage de chargement supérieur (330), chacune des parties (250, 350) pouvant coulisser de manière translatoire le long des pistes (242, 246), et d'un trajet arqué correspondant, à proximité de la paire de bras espacés (226, 228) et des pistes (342, 346), et d'un trajet arqué correspondant, à proximité de l'autre paire de bras espacés (326, 328), respectivement, avant l'attache, par rapport à la paroi (218) de sorte que la partie (250) puisse tourner autour d'un troisième axe (XR250), qui s'étend transversalement au premier axe (X3), et que l'autre partie (350) peut tourner autour d'un quatrième axe (XR350), qui s'étend transversalement au deuxième axe (X5),
la partie (250) et l'autre partie (350) pouvant pivoter par rapport à la paroi (218) par rapport au premier axe (X3) et au deuxième axe (X5), respectivement.

2. Construction spinale (212) selon la revendication 1, les cavités (230, 330) étant disposées dans une orientation côte à côte.

3. Construction spinale (212) selon la revendication 1 ou 2, la paroi (218) comportant une première surface (222) qui définit au moins une part (224) de la première cavité et une seconde surface (322) qui définit au moins une part (324) de la seconde cavité.

4. Construction spinale (212) selon l'une des revendications 1 à 3, la première cavité (230) se trouvant à proximité de la seconde cavité (330) et étant espacée de celle-ci.

5. Construction spinale (212) selon l'une des revendications 1 à 4, le corps (214) comportant une configuration de section transversale en forme de w.

6. Construction spinale (212) selon l'une des revendications 1 à 5, le corps (214) comportant un raccord.

7. Construction spinale (212) selon l'une des revendications 1 à 6, le corps (214) comportant un récepteur d'implant d'une fixation osseuse ayant un arbre conçu pour pénétrer dans les tissus, le récepteur d'implant étant conçu pour la disposition d'au moins une tige spinale.

8. Construction spinale (212) selon l'une des revendications 1 à 7, les cavités (230, 330) étant disposées dans une orientation relativement parallèle.

9. Construction spinale (212) selon l'une des revendications 1 à 8, comprenant en outre une première tige spinale (150) disposée dans la première cavité (230) et une seconde tige spinale (152) disposée dans la seconde cavité (330).

10. Construction spinale (212) selon l'une des revendications 1 à 9, les parties (250, 350) pouvant pivoter dans une plage d'environ +/- 0 à 30 degrés par rapport à la paroi (218).

11. Construction spinale (212) selon l'une des revendications 1 à 10, la partie (250, 350) définissant une première surface (74) conçue pour une prise coulissante avec le corps (214) et une seconde surface concave (76) conçue pour une prise avec une tige spinale.
